# EUROPÄISCHE PATENTANMELDUNG

(11) **EP 1 090 993 A1**
(43) Veröffentlichungstag der Anmeldung: **11.04.2001**
(21) Anmeldenummer: 00121159.8
(22) Anmeldetag: 29.09.2000
(51) Int. Cl.: C12N 15/31, C12P 13/08, C12P 13/04, C07K 14/34

(54) **Für das lrp-Gen codierende Nukleotidsequenzen**

(30) Priorität: 05.10.1999 DE 19947792
(71) Anmelder: Degussa-Hüls Aktiengesellschaft, 60287 Frankfurt am Main (DE)
(72) Erfinder: Möckel, Bettina, Dr., 40597 Düsseldorf (DE); Pfefferle, Walter, Dr., 33790 Halle (Westf.) (DE); Pühler, Alfred, Prof., 33739 Bielefeld (DE); Kalinowski, Jörn, Dr., 33615 Bielefeld (DE); Bathe, Brigitte, Dr., 33154 Salzkotten (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c),
und Verfahren zur fermentativen Herstellung von L-Aminosäuren unter Verstärkung oder Abschwächung des lrp-Gens.

SEQUENZPROTOKOLL

<110> Degussa-Hüls AG

<120> Neue für das lrp-Gen kodierende Nukleotidsequenzen

<130> 990109 BT

<140>
<141>

<160> 9

<170> PatentIn Ver. 2.1

<210> 1
<211> 715
<212> DNA
<213> Corynebacterium glutamicum

<220>
<221> CDS
<222> (151)..(612)
<223> lrp-Gen

<220>
<221> -35_signal
<222> (62)..(67)

<220>
<221> -10_signal
<222> (88)..(93)

<400> 1
gccgataacc tttatcatct ggttccaggg ctgccttgga tggcgacacc tccaggcttg 60

aatgaatctc ttgcgttttt tgcacactac aatcatcaca caattgccgg gtagttttgt 120

tgccagtttg cgcacctcaa ctaggctatt gtg caa tat atg aag cta gat tcc 174
Met Gln Tyr Met Lys Leu Asp Ser
1               5

att gat cgc gca att att gcg gag ctt agc gcg aat gcg cgc atc tca 222
Ile Asp Arg Ala Ile Ile Ala Glu Leu Ser Ala Asn Ala Arg Ile Ser
    10                  15                  20

aat ctc gca ctg gct gac aag gtg cat ctc act ccg gga cct tgc ttg 270
Asn Leu Ala Leu Ala Asp Lys Val His Leu Thr Pro Gly Pro Cys Leu
25                  30                  35                  40

agg agg gtg cag cgt ttg gaa gcc gaa gga atc att ttg ggc tac agc 318
Arg Arg Val Gln Arg Leu Glu Ala Glu Gly Ile Ile Leu Gly Tyr Ser
                45                  50                  55

gcg gac att cac cct gcg gtg atg aat cgt gga ttt gag gtg acc gtg 366
Ala Asp Ile His Pro Ala Val Met Asn Arg Gly Phe Glu Val Thr Val
            60                  65                  70

gat gtc act ctc agc aac ttc gac cgc tcc act gta gac aat ttt gaa 414
Asp Val Thr Leu Ser Asn Phe Asp Arg Ser Thr Val Asp Asn Phe Glu
        75                  80                  85

agc tcc gtt gcg cag cat gat gaa gta ctg gag ttg cac agg ctt ttt     462
Ser Ser Val Ala Gln His Asp Glu Val Leu Glu Leu His Arg Leu Phe
     90                  95                  100

ggt tcg cca gat tat ttt gtc cgc atc ggc gtt gct gat ttg gag gcg     510
Gly Ser Pro Asp Tyr Phe Val Arg Ile Gly Val Ala Asp Leu Glu Ala
105                 110                 115                 120

tat gag caa ttt tta tcc agt cac att caa acc gtg cca gga att gca     558
Tyr Glu Gln Phe Leu Ser Ser His Ile Gln Thr Val Pro Gly Ile Ala
                125                 130                 135

aag atc tca tca cgt ttt gct atg aaa gtg gtg aaa cca gct cgc ccc     606
Lys Ile Ser Ser Arg Phe Ala Met Lys Val Val Lys Pro Ala Arg Pro
            140                 145                 150

cag gtg tgaagcatgc attttgaagc atgaatcttt ttcatctagt gaaggactga     662
Gln Val

tcccatgcgt atgaaatcaa tcgcagcaat tgcaatcgct accgccgccc tgg          715


<210> 2
<211> 154
<212> PRT
<213> Corynebacterium glutamicum

<400> 2
Val Gln Tyr Met Lys Leu Asp Ser Ile Asp Arg Ala Ile Ile Ala Glu
  1               5                  10                  15

Leu Ser Ala Asn Ala Arg Ile Ser Asn Leu Ala Leu Ala Asp Lys Val
             20                  25                  30

His Leu Thr Pro Gly Pro Cys Leu Arg Arg Val Gln Arg Leu Glu Ala
         35                  40                  45

Glu Gly Ile Ile Leu Gly Tyr Ser Ala Asp Ile His Pro Ala Val Met
     50                  55                  60

Asn Arg Gly Phe Glu Val Thr Val Asp Val Thr Leu Ser Asn Phe Asp
 65                  70                  75                  80

Arg Ser Thr Val Asp Asn Phe Glu Ser Ser Val Ala Gln His Asp Glu
                 85                  90                  95

Val Leu Glu Leu His Arg Leu Phe Gly Ser Pro Asp Tyr Phe Val Arg
            100                 105                 110

Ile Gly Val Ala Asp Leu Glu Ala Tyr Glu Gln Phe Leu Ser Ser His
        115                 120                 125

Ile Gln Thr Val Pro Gly Ile Ala Lys Ile Ser Ser Arg Phe Ala Met
    130                 135                 140

Lys Val Val Lys Pro Ala Arg Pro Gln Val
145                 150


<210> 3
<211> 19
<212> DNA
<213> Corynebacterium glutamicum

<220>
<223> lrp_up_for

<400> 3
ggcgcccggg ggcgtgcgc                                                    19


<210> 4
<211> 48
<212> DNA
<213> Corynebacterium glutamicum

<220>
<223> lrp_up_rev

<400> 4
cccatccact aaacttaaac aaatggaatc tagcttcata tattgcac                    48


<210> 5
<211> 45
<212> DNA
<213> Corynebacterium glutamicum

<220>
<223> lrp_do_for

<400> 5
tgtttaagtt tagtggatgg ggctatgaaa gtggtgaaac cagct                       45


<210> 6
<211> 24
<212> DNA
<213> Corynebacterium glutamicum

<220>
<223> lrp_do_rev

<400> 6
ccggtctaga ggatccgcaa ttcc                                              24


<210> 7
<211> 397
<212> DNA
<213> Corynebacterium glutamicum

<220>
<223> lrp-Teil 1

<400> 7
ggcgcccggg ggcgtgcgcc cacaacgagg gcgatgacca gcatttcggt ggagcccgcg 60
aaaatcaggc cggaaaacag tggggctgcc caccattcgt agccgtattg aataaccaag 120
agaccaaacg caataccaat cgggtacatg cccaaacctg cagcaaggga ggtttttaga 180
ccttgcgcga tttcgtagcg ccgataacct ttatcatctg gttccagggc tgccttggat 240
ggcgacacct ccaggcttga atgaatctct tgcgtttttt gcacactaca atcatcacac 300
aattgccggg tagttttgtt gccagtttgc gcacctcaac taggctattg tgcaatatat 360
gaagctagat tccatttgtt taagtttagt ggatggg                                397


<210> 8
<211> 402
<212> DNA
<213> Corynebacterium glutamicum

<220>
<223> lrp-Teil 2

<400> 8
tgtttaagtt tagtggatgg ggctatgaaa gtggtgaaac cagctcgccc ccaggtgtga 60
agcatgcatt ttgaagcatg aatctttttc atctagtgaa ggactgatcc catgcgtatg 120
aaatcaatcg cagcaattgc aatcgctacc gccgccctgg ctggtggcac tggagtcgct 180
agcgcacagg aagctggctc gactgctggt tcctccaacc tcagctctgg gatccagctt 240
ccacaagaag ccaccgatct gttgagcttc ctgcctgctg ctcaggctgc gcaggttgaa 300
ggagcaattc agagcaccgc tggtttcttt gccgttggac tcggctcgac cgccattggt 360
tccactgcag tgaccttggg aattgcggat cctctagacc gg 402

<210> 9
<211> 778
<212> DNA
<213> Corynebacterium glutamicum

<400> 9
ggcgcccggg ggcgtgcgcc cacaacgagg gcgatgacca gcatttcggt ggagcccgcg 60
aaaatcaggc cggaaaacag tggggctgcc caccattcgt agccgtattg aataaccaag 120
agaccaaacg caataccaat cgggtacatg cccaaacctg cagcaaggga ggtttttaga 180
ccttgcgcga tttcgtagcg ccgataacct ttatcatctg gttccagggc tgccttggat 240
ggcgacacct ccaggcttga atgaatctct tgcgtttttt gcacactaca atcatcacac 300
aattgccggg tagttttgtt gccagtttgc gcacctcaac taggctattg tgcaatatat 360
gaagctagat tccatttgtt taagtttagt ggatggggct atgaaagtgg tgaaaccagc 420
tcgcccccag gtgtgaagca tgcattttga agcatgaatc tttttcatct agtgaaggac 480
tgatcccatg cgtatgaaat caatcgcagc aattgcaatc gctaccgccg ccctggctgg 540
tggcactgga gtcgctagcg cacaggaagc tggctcgact gctggttcct ccaacctcag 600
ctctgggatc cagcttccac aagaagccac cgatctgttg agcttcctgc ctgctgctca 660
ggctgcgcag gttgaaggag caattcagag caccgctggt ttctttgccg ttggactcgg 720
ctcgaccgcc attggttcca ctgcagtgac cttgggaatt gcggatcctc tagaccgg 778

## Beschreibung

Gegenstand der Erfindung sind für das lrp-Gen kodierende Nukleotidsequenzen und Verfahren zur fermentativen Herstellung von Aminosäuren, insbesondere Lysin und Isoleucin unter Verwendung von coryneformen Bakterien, in denen die Expression des lrp-Gens beeinflußt wird.

### Stand der Technik

L-Aminosäuren finden in der Tierernährung, in der Lebensmittelindustrie, in der Humanmedizin und in der pharmazeutischen Industrie Anwendung.

Es ist bekannt, daß diese Aminosäuren durch Fermentation von Stämmen coryneformer Bakterien insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Aminosäuren sind und L-Aminosäuren produzieren. Seit einigen Jahren werden ebenfalls Methoden der rekombinanten DNA-Technik zur Stammverbesserung L-Aminosäure produzierender Stämme von Corynebacterium eingesetzt LRP (leucine-responsive protein) ist ein zuerst für Escherichia coli beschriebener globaler Regulator, der die Transkription einer Reihe von Genen beeinflußt, deren Genprodukte am Transport, der Biosynthese und dem Abbau von Aminosäuren beteiligt sind (Calvo und Matthews, Microbiological Reviews 58, 466-490, 1994).

In den letzten Jahren wurden ähnliche Gene auch in anderen Organismen wie Bradyrhizobium japonicum (King und O'Brian, Journal of Bacteriology, 179, 1828-1831, 1997), Klebsiella aerogenes (Janes und Bender, Journal of Bacteriology, 181, 1054-1058, 1999), Sulfolobus acidocaldarius (Charlier et al., Gene, 201, 63-68, 1997) und in dem gram positiven Bakterium Bacillus subtilis (Belitsky et al., Journal of Bacteriology, 179, 5448-5457, 1997) identifiziert.

Das Lrp Protein reguliert in E. coli seine eigene Expression. Lrp beeinflußt außerdem in E. coli die Expression vieler Gene, entweder negativ oder positiv. Im allgemeinen wird dabei die Expression von Genprodukten, die in biosynthetischen Wegen wirksam sind, stimuliert. Genprodukte, die katabolisch wirksam sind werden in der Regel entsprechend negativ reguliert. In einigen Fällen potenziert sich die Wirkung von Lrp durch Zugabe von L-Leucin, während L-Leucin-Zugabe auch negativ wirken kann (Newman et al., In: Neidhardt et al. Escherichia coli and Salmonella typhimurium: Cellular and molecular biology. American Society for Microbiology, Washington D.C., 1513-1525, 1996). Durch LRP werden in E. coli eine große Zahl von Genen und Operons reguliert, die in der Aminosäurebiosynthese und dem Aminosäurekatabolismus eine zentrale Rolle spielen. In E. coli werden beispielsweise folgende Operons durch Lrp negativ reguliert: livJ, welches für ein Bindeprotein für ein hochaffines Aufnahmesystem von verzweigtkettigen Aminosäuren kodiert (Haney et al., Journal of Bacteriology, 174, 108-115, 1992) und lysU, daß für die Lysin tRNA Synthetase kodiert (Gazeau et al., FEBS Letters, 300, 254-258, 1994). Zu den bisher bekannten in E. coli positiv beeinflußten Genen gehören u.a. ilvIH, gltBDF und leuABCD (Lin et al., Journal of Bacteriology, 174, 1948-1955, 1992).

Letztgenanntes Operon ist von grundsätzlichem Interesse für die Leucinbiosynthese und von besonderem Interesse für die Lysinbiosynthese von Corynebacterium glutamicum. Es wird vermutet, daß eine Verbindung besteht zwischen Leucin-Auxotrophie und hohen Lysin-Produktivitäten (Schrumpf et al., Applied Microbiology and Biotechnology, 37, 566-571, 1992). Patek et al. (Applied and Environmental Microbiology, 60, 133-140, 1994) zeigten, daß eine Inaktivierung von leuA in einigen Lysinproduzenten von C. glutamicum zu erhöhten Lysinausbeuten führt. Tosaka et al. (Agricultural Biological Chemistry, 43, 265-270, 1979) konnten bei einer Mutante von Brevibacterium lactofermentum durch Zugabe von L-Leucin eine Erniedrigung der Lysinbildung bei gleichzeitiger Erhöhung der Threoninbildung erreichen.

### Aufgabe der Erfindung

Die Erfinder haben sich zur Aufgabe gestellt neue Maßnahmen zur verbesserten fermentativen Herstellung von L-Aminosäuren insbesondere L-Lysin und L-Isoleucin mit coryneformen Bakterien zur Verfügung zu stellen.

### Beschreibung der Erfindung

Gegenstand der Erfindung ist ein isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a), oder b) und
d) Polynukleotid, enthaltend mindestens 15 aufeinanderfolgende Basen der Polynukleotidsequenz von a), b) oder c).

Gegenstand der Erfindung ist ebenfalls das Polynukleotid gemäß Anspruch 1, wobei es sich bevorzugt um eine replizierbare DNA handelt, enthaltend:
(i) die Nukleotidsequenz, gezeigt in SEQ-ID-No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz i) oder (ii) komplementären Sequenz hybridisiert, und gegebenenfalls
(iv) funktionsneutralen Sinnmutationen in (i).

Weitere Gegenstände sind
ein Polynukleotid gemäß Anspruch 2, enthaltend die Nukleotidsequenz wie in SEQ ID No. 1 dargestellt,
ein Polynukleotid gemäß Anspruch 2, das für ein Polypeptid codiert, das die Aminosäuresequenz, wie in SEQ ID No. 2 dargestellt, enthält.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthält, mit einer Sonde, die die Sequenz des genannten Polynukleotids gemäss SEQ ID No. 1 oder ein Fragment davon enthält und Isolierung der genannten DNA-Sequenz.

Polynukleotidsequenzen gemäss der Erfindung sind geeignet als Hybridisierungs-Sonden für RNA, cDNA und DNA, um cDNA in voller Länge zu isolieren, die für Lrp-Protein codieren und solche cDNA oder Gene zu isolieren, die eine hohe Ähnlichkeit der Sequenz mit der des lrp Gens aufweisen.

Polynukleotidsequenzen gemäss der Erfindung sind weiterhin geeignet als Primer zur Herstellung von DNA von Genen, die für Lrp-Proteine codieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Basen. Geeignet sind ebenfalls Oligonukleotide mit einer Länge von mindestens 40 oder 50 Basenpaaren.

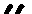 Isoliert" bedeutet aus seinem natürlichen Umfeld herausgetrennt.

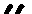 Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter 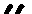 Polypeptiden" versteht man Peptide oder Proteine, die zwei oder mehr über Peptidbindungen verbundene Aminosäuren enthalten.

Die Polypeptide gemäß Erfindung schliessen ein Polypeptid gemäß SEQ ID No. 2, insbesondere solche mit der biologischen Aktivität der Lrp-Proteins und auch solche ein, die zu wenigstens 70 % identisch sind mit dem Polypeptid gemäss SEQ ID No. 2, bevorzugt zu wenigstens 80% und besonders die zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von Aminosäuren insbesondere L-Lysin und L-Isoleucin mittels coryneformer Bakterien, die insbesondere bereits Aminosäuren produzieren, indem man das neue lrp-Gen in Abhängigkeit von der Zielsubstanz verstärkt oder abschwächt.

Der Begriff 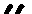 Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promoter verwendet oder ein Gen verwendet, das für ein entsprechendes Enzym mit einer hohen Aktivität kodiert und gegebenenfalls diese Maßnahmen kombiniert.

Der Begriff 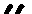 Abschwächung" beschreibt in diesem Zusammenhang die Verringerung oder Ausschaltung der intrazellulären Aktivität eines oder mehrere Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Enzym (Protein) inaktiviert und gegebenenfalls diese Maßnahmen kombiniert.

Die Mikroorganismen, die Gegenstand der vorliegenden Erfindung sind, können L-Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind zum Beispiel die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte Aminosäure produzierende Mutanten bzw. Stämme, wie beispielsweise
die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715
oder die L-Isoleucin produzierenden Stämme
Corynebacterium glutamicum ATCC 14309
Corynebacterium glutamicum ATCC 14310
Corynebacterium glutamicum ATCC 14311
Corynebacterium glutamicum ATCC 15168 und
Corynebacterium ammoniagenes ATCC 6871.

Den Erfindern gelang es, das neue, für das Lrp-Protein kodierende lrp-Gen von C. glutamicum zu isolieren.

Zur Isolierung des lrp-Gens oder auch anderer Gene von C. glutamicum wird zunächst eine Genbank dieses Mikrorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 (Raleigh et al., 1988, Nucleic Acids Research 16:1563-1575) angelegt wurde. Börmann et al. (Molecular Microbiology 6(3), 317-326)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Viera et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige für die Sequenzierung geeignete Vektoren subkloniert und anschließend sequenziert werden, so wie es z.B. bei Sanger et al. (Proceedings of the National Academy of Sciences of the United States of America, 74:5463-5467, 1977) beschrieben ist.

Auf diese Weise wurde die neue für das Gen lrp kodierende DNA-Sequenz von C. glutamicum erhalten, die als SEQ ID NO 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID NO 2 ist die sich ergebende Aminosäuresequenz des lrp-Genproduktes dargestellt.

Kodierende DNA-Sequenzen, die sich aus SEQ ID NO 1 durch die Degeneriertheit des genetischen Codes ergeben, sind ebenfalls Bestandteil der Erfindung. In gleicher Weise sind DNA-Sequenzen, die mit SEQ ID NO 1 oder Teilen von SEQ ID NO 1 hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als 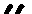 Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2 ergeben, sind ebenfalls Bestandteil der Erfindung.

In gleicher Weise sind DNA-Sequenzen, die mit SEQ ID NO 1 oder Teilen von SEQ ID NO 1 hybridisieren, Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID NO 1 ergeben. Derartige Oligonukleotide haben typischerweise eine Länge von mindestens 15 Basenpaaren.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß das lrp-Gens wichtig für die Herstellung von Aminosäuren, insbesondere L-Lysin und L-Isoleucin ist.

Die Erfinder fanden weiterhin heraus, daß die zusätzliche Überexpression ein oder mehrerer Enzyme des jeweiligen Biosyntheseweges, der Glykolyse, des Zitronensäurezyklus, des anaplerotischen Stoffwechsels oder des Aminosäure-Exportes einzeln oder in Kombination miteinander weitere Vorteile für die Herstellung von Aminosäuren, insbesondere L-Lysin und L-Isoleucin ergibt.

So kann beispielsweise für die Herstellung von L-Lysin
- gleichzeitig das für die Dihydrodipicolinat-Synthase kodierende dapA-Gen (EP-B 0 197 335), oder
- gleichzeitig das für die Glycerinaldehyd-3-Phosphat-Dehydrogenase kodierende gap-Gen (Schwinde et al., Journal of Bacteriology 175: 3905-3908 (1993)), oder
- gleichzeitig das für die Malat:Chinon Oxidoreduktase kodierende mqo-Gen (Molenaar et al., European Journal of Biochemistry 254, 395 - 403 (1998)), oder
- gleichzeitig das für die Pyruvat-Carboxylase kodierende pyc-Gen (DE-A-19 831 609), oder
- gleichzeitig das für den Lysin-Export kodierende LysE-Gen (DE-A-195 48 222)
überexprimiert werden.

So kann beispielsweise für die Herstellung von L-Isoleucin
- gleichzeitig das für die Threonin-Dehydratase kodierende ilvA-Gen (Möckel et al., Journal of Bacteriology (1992) 8065-8072)) oder das für eine "feed back resistente" Threonin- Dehydratase kodierende ilvA(Fbr)-Allel (Möckel et al., (1994)Molecular Microbiology 13: 833-842), oder
- gleichzeitig das für die Glycerinaldehyd-3-Phosphat-Dehydrogenase kodierende gap-Gen (Schwinde et al., Journal of Bacteriology 175: 3905-3908 (1993)), oder
- gleichzeitig das für die Malat:Chinon Oxidoreduktase kodierende mqo-Gen (Molenaar et al., European Journal of Biochemistry 254, 395 - 403 (1998)), oder
- gleichzeitig das für die Pyruvat-Carboxylase kodierende pyc-Gen (DE-A-19 831 609)
überexprimiert werden.

Weiterhin kann es für die Produktion von Aminosäuren insbesondere L-Lysin und L-Isoleucin vorteilhaft sein unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren, insbesondere L-Lysin und L-Isoleucin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoffhaltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum der gewünschten Aminosäure gebildet hat. Dieses Ziel wird normalerweise innerhalb von 10 Stunden bis 160 Stunden erreicht.

Die Analyse der Aminosäuren kann durch Anionenaustauschchromatographie mit anschließender Ninhydrin Derivatisierung erfolgen, so wie bei Spackman et al. (Analytical Chemistry, 30, (1958), 1190) beschrieben, oder sie kann durch reversed phase HPLC erfolgen so wie bei Lindroth et al. (Analytical Chemistry (1979) 51: 1167-1174) beschrieben.

Das erfindungsgemäße Verfahren dient zur fermentativen Herstellung von Aminosäuren, insbesondere L-Lysin und L-Isoleucin.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al., (1995, Plasmid 33:168-179) beschrieben, isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Res. 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A laboratory Manual, Cold Spring Harbor) beschrieben durchgeführt, wobei die Zellen auf LB-Agar (Lennox, 1955, Virology, 1:190) + 100µg/ml Ampicillin ausplattiert wurden. Nach Inkubation über Nacht bei 37°C wurden rekombinante Einzelklone selektioniert.

### Beispiel 2

### Isolierung und Sequenzierung des lrp-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größembereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) + 50 µg/ml Zeocin ausplattiert. Die Plasmidpräparation der rekombinanten Klone erfolgte mit dem Biorobot 9600 (Product No. 900200, Qiagen, Hilden, Germany). Die Sequenzierung erfolgte nach der Dideoxy-Kettenabbruch-Methode von Sanger et al. (1977, Proceedings of the National Academies of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Germany) angewandt. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Germany).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant NCBI-Datenbank der "National Library of Medicine" (USA) durchgeführt.

Die erhaltene Nukleotidsequenz ist in SEQ ID NO 1. Die Analyse der Nukleotidsequenz ergab ein offenes Leseraster von 462 Basenpaaren, welches als lrp-Gen bezeichnet wurde. Das lrp-Gen kodiert für ein Polypeptid von 154 Aminosäuren.

### Beispiel 3

### Einbau einer Deletion/Insertion in das lrp-Gen

Hierzu wurde aus dem Stamm ATCC13032 nach der Methode von Tauch et al. (1995, Plasmid 33:168-179) chromosomale DNA isoliert. Aufgrund der aus Beispiel 2 für C.glutamicum bekannten Sequenz des lrp-Gens wurden die im folgenden beschriebenen Oligonukleotide für die Erzeugung des lrp Deletionsallels ausgewählt.
lrp-up-for (Siehe auch SEQ ID No.3)
   5*'*-GGC G(CC CGG) GGG CGT GCG C-3'
lrp_up_rev (Siehe auch SEQ ID No.4 )
   5*'*-Extension-CCC ATC CAC TAA ACT TAA ACA-Seite-AAT GGA ATC TAG CTT CAT ATA TTG CAC-3'
lrp_do_for: (Siehe auch SEQ ID No. 5)
   5*'*-Extension-TGT TTA AGT TTA GTG GAT GGG-Seite-GCT ATG AAA GTG GTG AAA CCA GCT-3'
lrp_do_rev (Siehe auch SEQ ID No. 6)
   5*'*-CCG G(TC TAG) AGG ATC CGC AAT TCC-3'

Die dargestellten Primer wurden von der Firma MWG Biotech (Ebersberg, Deutschland) synthetisiert und die PCR-Reaktion unter Verwendung der Pfu-Polymerase (Stratagene, Product. No. 600135, La Jolla, USA) und des PTC 100-Thermocyclers (MJ Research Inc., Waltham, USA) durchgeführt. Die Primer lrp_up_for und lrp_do_rev enthalten eingefügt eine XmaI (lrp_up_for) bzw. eine XbaI (lrp_do_rev) Schnittstelle, die in der Darstellung in Klammern angegeben sind.
Das erste 397 bp DNA-Amplifikat, als lrp-Teil 1 bezeichnet, wurde mit den Oligonukleotiden lrp_up_for und lrp_up_rev erhalten. Es beinhaltet den 5*'*-Bereich des lrp-Gens (Nukleotid 1-18), den 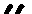 upstream" Bereich des Gens mit 332 Nukleotiden und zusätzlich die vom Oligonukleotid lrp_up_rev stammende 21 bp-Extension, die komplementär ist zu dem 5*'*-Ende des Primers lrp_do_for.

Zur Sequenzkontrolle erfolgte die Sequenzierung des Fragmentes lrp-Teil 1 nach der Didesoxy-Kettenabbruch-Methode, wie es von Sanger et al. (Proceedings of the National Academy of Sciences of the United States of America, 74:5463-5467, 1977) beschrieben ist. Die ermittelte Sequenz ist in SEQ ID No.7 dargestellt.

Das zweite 402 bp DNA-Fragment, lrp-Teil 2, wurde mit den Oligonukleotiden lrp_do_for und lrp_do_rev erhalten und beinhaltet die 3*'*-Region des lrp-Gens (Nukleotid 426-462), und 366 bp 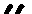 downstream" des Gens.

Dieses wurde ebenso wie bei Sanger et al. beschrieben (Proceedings of the National Academy of Sciences of the United States of America, 74:5463-5467, 1977) sequenziert. Die ermittelte Sequenz ist in SEQ ID No.8 gezeigt.

Die beiden PCR-Produkte lrp-Teil 1 und lrp-Teil 2 wurden in einer dritten PCR-Reaktion gemeinsam als DNA-Template verwendet, wobei sich aufgrund des überlappenden, komlementären DNA-Bereiches der 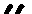 Hinstrang" von lrp-Teil 1 mit dem 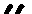 Rückstrang" von lrp-Teil 2 verbinden konnte. Die Extension dieses überlappenden DNA-Bereiches bzw. die Zugabe der Oligonukleotide lrp_up_for und lrp_do_rev, führt zur Erzeugung eines 778 bp PCR-Amplifikats, welches zur Erzeugung eines lrp-Deletionsderivates führt, wie dargestellt in SEQ ID No. 9.

### Beispiel 4

### Einbau des Δlrp-Allels in das Chromosom

Das in Beispiel 3 erhaltene 778 bp große lrp-Deletionsallel, wurde mittels Deletionsmutagenese unter Zuhilfenahme des bei Schäfer et al., Gene, 14, 69-73 (1994) beschriebenen sacB-Systems in das Chromosom von C. glutamicum eingebaut. Dieses System erlaubt dem Fachmann die Identifizierung bzw. die Selektion von Allel-Austauschen, die sich durch homologe Rekombination vollziehen.

### 1. Konstruktion des Austauschvektors pK19mobsacBΔlrp

Das in Beispiel 3 erhaltene lrp-Deletionsderivat entsprechend SEQ ID NO. 9, wurde mit den Restriktionsenzymen XmaI- und XbaI (Amersham-Pharmarcia, Freiburg, Germany) geschnitten ,nach Auftrennung in einem Agarosegel (0,8%) mit Hilfe des Qiagenquick Gel Extraction Kit (Qiagen, Hilden, Germany) aus dem Agarosegel isoliert und mit dem bei Schäfer et al., Gene, 14, 69-73 (1994) mobilisierbaren Klonierungsvektor pK19mobsacB zur Ligation eingesetzt. Dieser wurde zuvor mit dem Restriktionsenzymen XmaI- und XbaI aufgespalten, mit dem lrp- Deletionsallel gemischt und mit T4-DNA-Ligase (Amersham-Pharmaecia, Freiburg, Germany) behandelt.
Anschließend wurde der E.coli Stamm S17-1 (Simon et al.,Bio/Technologie 1: 784-791, 1993) mit dem Ligationsansatz (Hanahan, In. DNA cloning. A practical approach. Vol.1. ILR-Press, Cold Spring Habor, New York, 1989) elektroporiert. Die Selektion der Plasmid-tragenden Zellen erfolgte durch Ausplattieren des Tansformationsansatztes auf LB Agar (Sambrock et al., Molecular Cloning: a laboratory manuel. 2^{nd} Ed. Cold Spring Habor, New York, 1989), der mit 25 mg/l Kanamycin supplementiert worden war.
Plasmid-DNA wurde aus einer Transformante mit Hilfe des QIAprep Spin Miniprep Kit der Firma Qiagen isoliert und das klonierte lrp-Deletionsallel mittels Sequenzierung durch die Firma MWG Biotech (Ebersberg, Deutschland) verifiziert. Das Plasmid wurde pK19mobscBΔlrp genannt. Der Stamm wurde als E. coli S17-1/pK19mobsacBΔlrp unter der Nummer DSM 13702 am 31.08.2000 bei der deutschen Sammlung für Mikrooorganismen und Zellkulturen (DSMZ, Braunschweig, Deutschland) hinterlegt.

### Beispiel 5

### Deltionsmutagenese des lrp-Gens in dem C. glutamicum Stamm DSM 5715

Der in Beispiel 4 genannte Vektor pK19mobsacBΔlrp wurde nach der Elekroporationsmethode von Tauch et al.,(1989 FEMS Microbiology Letters 123: 343-347) elekroporiert. Der Vektor kann in DSM5715 nicht selbständig replizieren und bleibt nur dann in der Zelle erhalten, wenn er ins Chromosom integriert hat. Die Selektion von Klonen mit integriertem pK19mobsacBΔlrp erfolgte durch Ausplattieren des Elekroporationsansatzes auf LB-Agar (Sambrock et al., Molecular Cloning: a laboratory manuel. 2^{nd} Ed. Cold Spring Habor, New York, 1989), der mit 15 mg/l Kanamycin supplementiert worden war. Angewachsene Klone wurden auf LB-Agarplatten mit 25 mg/l Kanamycin ausgestrichen und für 16 Stunden bei 33°C inkubiert. Um die Excision des Plasmides zusammen mit der vollständigen chromosomalen Kopie des lrp-Gens zu erreichen, wurden die Klone anschließend auf LB-Agar mit 10% Sucrose angezogen. Das Plasmid pK19mobsacB enthält eine Kopie des sacB-Gens, welches Sucrose in die für C. glutamicum toxische Levansucrase umwandelt. Auf LB-Agar mit Sucrose wachsen daher nur solche Klone an, bei denen das integrierte pK19mobsacBΔlrp wiederum excisiert hat. Bei der Excision kann zusammen mit dem Plasmid entweder die vollständige chromosomale Kopie des lrp-Gens excisieren, oder die unvollständige Kopie mit der internen Deletion. Um nachzuweisen, daß die unvollständige Kopie von lrp im Chromosom verblieben ist, wurde das Plasmid pK9mobsacBΔlrp Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert. Chromosomale DNA einer potentiellen Deletionsmutante wurde nach der Methode von Eikmanns et al. (Microbiology 140: 1817-1828 (1994)) isoliert und jeweils mit dem Restriktionsenzymen SphI und PstI in getrennten Ansätzen geschnitten. Die entstehenden Fragmente wurden mit Agarosegel-Elektrophorese aufgetrennt und mit dem Dig-Hybrisierungskit der Firma Boehringer bei 68°C hybridisiert. Bei dem Kontrollstamm wurden zwei hybridisierende Fragmente von ca. 1640bp und 960 bp erhalten, während bei der Mutante zwei hybridisierende Fragmente von ca. 580 bp und 1300 bp erhalten wurden. Damit konnte gezeigt werden, daß der Stamm DSM5715 seine vollständige Kopie des lrp-Gens verloren hat und stattdessen nur noch über die unvollständige Kopie mit der Deletion von ca. 380 bp verfügt.

Der Stamm wurde als C. glutamicum DSM5715/pK19mobsacBΔlrp bezeichnet.

### Beispiel 6

### Herstellung von Lysin

Der in Beispiel 5 erhaltene C. glutamicum Stamm DSM5715/pK19mobsacBΔlrp wurde in einem zur Produktion von Lysin geeigneten Nährmedium kultiviert und der Lysingehalt im Kulturüberstand bestimmt.

Dazu wurde der Stamm zunächst auf Agarplatte für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Die Vorkultur wurde 48 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 OD betrug. Für die Hauptkultur wurde das Medium MM verwendet.

| Medium MM | |
|---|---|
| CSL (Corn Steep Liquor) | 5 g/l |
| MOPS | 20 g/l |
| Glucose (getrennt autoklaviert) | 50g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃ zugesetzt.

Die Kultivierung erfolgte in 10 ml Volumen in einem 100 ml Erlenmeyerkolben mit Schikanen. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuches dargestellt.

**Tabelle 1**

| Stamm | OD(660) | Lysin-HCl g/l |
|---|---|---|
| DSM5715 | 7,9 | 13,53 |
| DSM5715/pK19mobsacBΔlrp | 7,8 | 14,27 |

### Beispiel 3

### Einbau einer Deletion/Insertion in das lrp-Gen

Hierzu wurde aus dem Stamm ATCC13032 nach der Methode von Tauch et al. (1995, Plasmid 33:168-179) chromosomale DNA isoliert. Aufgrund der aus Beispiel 2 für C.glutamicum bekannten Sequenz des lrp-Gens wurden die im folgenden beschriebenen Oligonukleotide für die Erzeugung des lrp Deletionsallels ausgewählt.
lrp-up-for (Siehe auch SEQ ID No.3)
   5*'*-GGC G(CC CGG) GGG CGT GCG C-3'
lrp_up_rev (Siehe auch SEQ ID No.4 )
   5*'*-Extension-CCC ATC CAC TAA ACT TAA ACA-Seite-AAT GGA ATC TAG CTT CAT ATA TTG CAC-3'
lrp_do_for: (Siehe auch SEQ ID No. 5)
   5*'*-Extension-TGT TTA AGT TTA GTG GAT GGG-Seite-GCT ATG AAA GTG GTG AAA CCA GCT-3'
lrp_do_rev (Siehe auch SEQ ID No. 6)
   5*'*-CCG G(TC TAG) AGG ATC CGC AAT TCC-3'

Die dargestellten Primer wurden von der Firma MWG Biotech (Ebersberg, Deutschland) synthetisiert und die PCR-Reaktion unter Verwendung der Pfu-Polymerase (Stratagene, Product. No. 600135, La Jolla, USA) und des PTC 100-Thermocyclers (MJ Research Inc., Waltham, USA) durchgeführt. Die Primer lrp_up_for und lrp_do_rev enthalten eingefügt eine XmaI (lrp_up_for) bzw. eine XbaI (lrp_do_rev) Schnittstelle, die in der Darstellung in Klammern angegeben sind.

Das erste 397 bp DNA-Amplifikat, als lrp-Teil 1 bezeichnet, wurde mit den Oligonukleotiden lrp_up_for und lrp_up_rev erhalten. Es beinhaltet den 5*'*-Bereich des lrp-Gens (Nukleotid 1-18), den 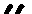 upstream" Bereich des Gens mit 332 Nukleotiden und zusätzlich die vom Oligonukleotid lrp_up_rev stammende 21 bp-Extension, die komplementär ist zu dem 5*'*-Ende des Primers lrp_do_for.

Zur Sequenzkontrolle erfolgte die Sequenzierung des Fragmentes lrp-Teil 1 nach der Didesoxy-Kettenabbruch-Methode, wie es von Sanger et al. (Proceedings of the National Academy of Sciences of the United States of America, 74:5463-5467, 1977) beschrieben ist. Die ermittelte Sequenz ist in SEQ ID No.7 dargestellt.

Das zweite 402 bp DNA-Fragment, lrp-Teil 2, wurde mit den Oligonukleotiden lrp_do_for und lrp_do_rev erhalten und beinhaltet die 3*'*-Region des lrp-Gens (Nukleotid 426-462), und 366 bp 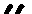 downstream" des Gens.

Dieses wurde ebenso wie bei Sanger et al. beschrieben (Proceedings of the National Academy of Sciences of the United States of America, 74:5463-5467, 1977) sequenziert. Die ermittelte Sequenz ist in SEQ ID No.8 gezeigt.

Die beiden PCR-Produkte lrp-Teil 1 und lrp-Teil 2 wurden in einer dritten PCR-Reaktion gemeinsam als DNA-Template verwendet, wobei sich aufgrund des überlappenden, komlementären DNA-Bereiches der 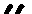 Hinstrang" von lrp-Teil 1 mit dem 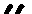 Rückstrang" von lrp-Teil 2 verbinden konnte. Die Extension dieses überlappenden DNA-Bereiches bzw. die Zugabe der Oligonukleotide lrp_up_for und lrp_do_rev, führt zur Erzeugung eines 778 bp PCR-Amplifikats, welches zur Erzeugung eines lrp-Deletionsderivates führt, wie dargestellt in SEQ ID No. 9.

### Beispiel 4

### Einbau des Δlrp-Allels in das Chromosom

Das in Beispiel 3 erhaltene 778 bp große lrp-Deletionsallel, wurde mittels Deletionsmutagenese unter Zuhilfenahme des bei Schäfer et al., Gene, 14, 69-73 (1994) beschriebenen sacB-Systems in das Chromosom von C. glutamicum eingebaut. Dieses System erlaubt dem Fachmann die Identifizierung bzw. die Selektion von Allel-Austauschen, die sich durch homologe Rekombination vollziehen.

### 2. Konstruktion des Austauschvektors pK19mobsacBΔlrp

Das in Beispiel 3 erhaltene lrp-Deletionsderivat entsprechend SEQ ID NO. 9, wurde mit den Restriktionsenzymen XmaI- und XbaI (Amersham-Pharmarcia, Freiburg, Germany) geschnitten ,nach Auftrennung in einem Agarosegel (0,8%) mit Hilfe des Qiagenquick Gel Extraction Kit (Qiagen, Hilden, Germany) aus dem Agarosegel isoliert und mit dem bei Schäfer et al., Gene, 14, 69-73 (1994) mobilisierbaren Klonierungsvektor pK19mobsacB zur Ligation eingesetzt. Dieser wurde zuvor mit dem Restriktionsenzymen XmaI- und XbaI aufgespalten, mit dem lrp- Deletionsallel gemischt und mit T4-DNA-Ligase (Amersham-Pharmaecia, Freiburg, Germany) behandelt.
Anschließend wurde der E.coli Stamm S17-1 (Simon et al.,Bio/Technologie 1: 784-791, 1993) mit dem Ligationsansatz (Hanahan, In. DNA cloning. A practical approach. Vol.1. ILR-Press, Cold Spring Habor, New York, 1989) elektroporiert. Die Selektion der Plasmid-tragenden Zellen erfolgte durch Ausplattieren des Tansformationsansatztes auf LB Agar (Sambrock et al., Molecular Cloning: a laboratory manuel. 2^{nd} Ed. Cold Spring Habor, New York, 1989), der mit 25 mg/l Kanamycin supplementiert worden war.
Plasmid-DNA wurde aus einer Transformante mit Hilfe des QIAprep Spin Miniprep Kit der Firma Qiagen isoliert und das klonierte lrp-Deletionsallel mittels Sequenzierung durch die Firma MWG Biotech (Ebersberg, Deutschland) verifiziert. Das Plasmid wurde pK19mobscBΔlrp genannt. Der Stamm wurde als E. coli S17-1/pK19mobsacBΔlrp unter der Nummer DSM 13702 am 31.08.2000 bei der deutschen Sammlung für Mikrooorganismen und Zellkulturen (DSMZ, Braunschweig, Deutschland) hinterlegt.

### Beispiel 5

### Deltionsmutagenese des lrp-Gens in dem C. glutamicum Stamm DSM 5715

Der in Beispiel 4 genannte Vektor pK19mobsacBΔlrp wurde nach der Elekroporationsmethode von Tauch et al., (1989 FEMS Microbiology Letters 123: 343-347) elekroporiert. Der Vektor kann in DSM5715 nicht selbständig replizieren und bleibt nur dann in der Zelle erhalten, wenn er ins Chromosom integriert hat. Die Selektion von Klonen mit integriertem pK19mobsacBΔlrp erfolgte durch Ausplattieren des Elekroporationsansatzes auf LB-Agar (Sambrock et al., Molecular Cloning: a laboratory manuel. 2^{nd} Ed. Cold Spring Habor, New York, 1989), der mit 15 mg/l Kanamycin supplementiert worden war. Angewachsene Klone wurden auf LB-Agarplatten mit 25 mg/l Kanamycin ausgestrichen und für 16 Stunden bei 33°C inkubiert. Um die Excision des Plasmides zusammen mit der vollständigen chromosomalen Kopie des lrp-Gens zu erreichen, wurden die Klone anschließend auf LB-Agar mit 10% Sucrose angezogen. Das Plasmid pK19mobsacB enthält eine Kopie des sacB-Gens, welches Sucrose in die für C. glutamicum toxische Levansucrase umwandelt. Auf LB-Agar mit Sucrose wachsen daher nur solche Klone an, bei denen das integrierte pK19mobsacBΔlrp wiederum excisiert hat. Bei der Excision kann zusammen mit dem Plasmid entweder die vollständige chromosomale Kopie des lrp-Gens excisieren, oder die unvollständige Kopie mit der internen Deletion. Um nachzuweisen, daß die unvollständige Kopie von lrp im Chromosom verblieben ist, wurde das Plasmid pK9mobsacBΔlrp Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert.

Chromosomale DNA einer potentiellen Deletionsmutante wurde nach der Methode von Eikmanns et al. (Microbiology 140: 1817-1828 (1994)) isoliert und jeweils mit dem Restriktionsenzymen SphI und PstI in getrennten Ansätzen geschnitten. Die entstehenden Fragmente wurden mit Agarosegel-Elektrophorese aufgetrennt und mit dem Dig-Hybrisierungskit der Firma Boehringer bei 68°C hybridisiert. Bei dem Kontrollstamm wurden zwei hybridisierende Fragmente von ca. 1640bp und 960 bp erhalten, während bei der Mutante zwei hybridisierende Fragmente von ca. 580 bp und 1300 bp erhalten wurden. Damit konnte gezeigt werden, daß der Stamm DSM5715 seine vollständige Kopie des lrp-Gens verloren hat und stattdessen nur noch über die unvollständige Kopie mit der Deletion von ca. 380 bp verfügt.
Der Stamm wurde als C. glutamicum DSM5715/pK19mobsacBΔlrp bezeichnet.

### Beispiel 6

### Herstellung von Lysin

Der in Beispiel 5 erhaltene C. glutamicum Stamm DSM5715/pK19mobsacBΔlrp wurde in einem zur Produktion von Lysin geeigneten Nährmedium kultiviert und der Lysingehalt im Kulturüberstand bestimmt.

Dazu wurde der Stamm zunächst auf Agarplatte für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Die Vorkultur wurde 48 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 OD betrug. Für die Hauptkultur wurde das Medium MM verwendet.

| Medium MM | |
|---|---|
| CSL (Corn Steep Liquor) | 5 g/l |
| MOPS | 20 g/l |
| Glucose (getrennt autoklaviert) | 50g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃ zugesetzt.

Die Kultivierung erfolgte in 10 ml Volumen in einem 100 ml Erlenmeyerkolben mit Schikanen. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuches dargestellt.

**Tabelle 1**

| Stamm | OD(660) | Lysin-HCl g/l |
|---|---|---|
| DSM5715 | 7,9 | 13,53 |
| DSM5715/pK19mobsacBΔlrp | 7,8 | 14,27 |

Folgende Figuren sind beigefügt:

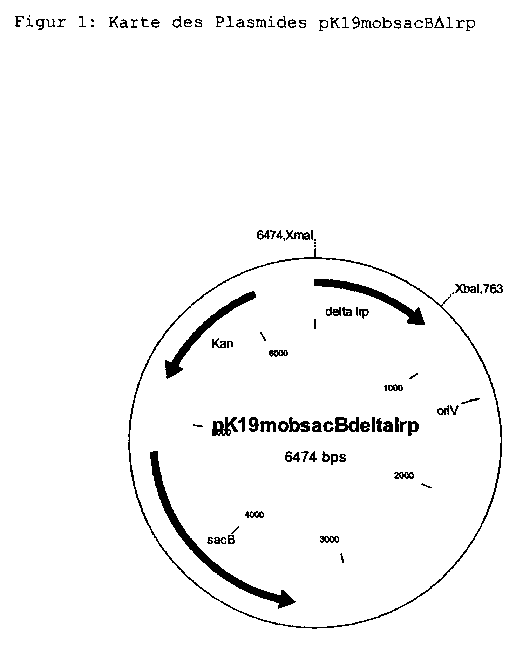

Figur 1: Karte des Plasmids pK19mobsacBΔlrp.

Die verwendeten Abkürzungen und Bezeichnungen haben folgende Bedeutung. Längenangaben sind als ca.-Werte aufzufassen.
- sacB:: sacB-Gen
- oriV:: Replikationsursprung V
- KmR:: Kanamycin Resistenz
- XmaI: Schnittstelle des Restriktionsenzyms XmaI
- XbaI: Schnittstelle des Restriktionsenzyms XbaI
- Δlrp:: unvollständiges Fragment des lrp-Gens mit interner 380 bp Deletion

## Patentansprüche

1. Isoliertes Polynukleotid aus coryneformen
Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c).

2. Polynukleotid gemäß Anspruch 1,
wobei das Polynukleotid eine in coryneformen Bakterien replizierbare, bevorzugt rekombinante DNA ist.

3. Polynukleotid gemäß Anspruch 1,
wobei das Polynukleotid eine RNA ist.

4. Polynukleotid gemäß Anspruch 2,
enthaltend die Nukleinsäuresequenz wie in SEQ ID No. 1 dargestellt.

5. Replizierbare DNA gemäß Anspruch 2, enthaltend
(i) die Nukleotidsequenz, gezeigt in SEQ-ID-No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

6. Polynukleotidsequenz gemäß Anspruch 2, das
für ein Polypeptid codiert, das die Aminosäuresequenz in SEQ ID No. 2 darstellt, enthält.

7. Verfahren zur Herstellung von L-Aminosäuren,
**dadurch gekennzeichnet**,
daß man folgende Schritte durchführt:
a) Fermentation der die gewünschte L-Aminosäure produzierenden Bakterien, in denen man zumindest das lrp Gen abschwächt oder verstärkt.
b) Anreicherung des gewünschten Produkts im Medium oder in den Zellen der Bakterien, und
c) Isolieren der L-Aminosäure.

8. Verfahren gemäß Anspruch 7,
**dadurch gekennzeichnet**,
daß man Bakterien einsetzt, in denen man zusätzlich weitere Gene des Biosyntheseweges der gewünschten L-Aminosäure verstärkt.

9. Verfahren gemäß Anspruch 7,
**dadurch gekennzeichnet**,
daß man Bakterien einsetzt, in denen die Stoffwechselwege zumindest teilweise ausgeschaltet sind, die die Bildung der gewünschten L-Aminosäure verringern.

10. Verfahren gemäß Anspruch 7,
**dadurch gekennzeichnet**,
daß man einen mit einem Plasmidvektor transformierten Stamm einsetzt, und der Plasmidvektor die für das lrp-Gen codierende Nukleotidsequenz trägt.

11. Verfahren gemäß Anspruch 7,
**dadurch gekennzeichnet**,
daß man in das lrp-Gen des Bakeriums eine Deletion oder Insertion zur Abschwächung einführt.

12. Verfahren gemäß einem oder mehreren der Ansprüche 8 bis 11,
**dadurch gekennzeichnet**,
daß man coryneforme Bakterien verwendet, die L-Aminosäure herstellen.
